# EUROPÄISCHE PATENTSCHRIFT

(11) **EP 2 302 002 B1**
(45) Veröffentlichungstag und Bekanntmachung des Hinweises auf die Patenterteilung: **27.03.2013**
(21) Anmeldenummer: 10173018.2
(22) Anmeldetag: 17.08.2010
(51) Int. Cl.: C09B 23/14, C09B 57/10, G01N 33/58

(54) **Difluoroboradiazaindacen-Farbstoffe**
Difluoroboradiazaindacene dyes
Colorant au difluoroboradiazaindacène

(30) Priorität: 28.08.2009 DE 102009028982
(43) Veröffentlichungstag der Anmeldung: 30.03.2011
(73) Patentinhaber: BAM Bundesanstalt für Materialforschung und -prüfung, 12205 Berlin (DE)
(72) Erfinder: Descalzo, Ana, B., Dr., 28007 Madrid (ES); Fischer, Tobias, 10827 Berlin (DE); Behnke, Thomas, 13349 Berlin (DE); Rurack, Knut, Dr., 12101 Berlin (DE)
(74) Vertreter: Zimmermann & Partner

(56) Entgegenhaltungen:
- WO-A2-2008/040994
- ZIESSEL ET AL.: "Solid-State Gas Sensors Developed from Functional Difluoroboradiazaindacene Dyes", CHEM. EUR. J., Bd. 15, Nr. 6, 20. September 2001 (2001-09-20), Seiten 1359-1369, XP002621773, DOI: 10.1002/chem.200801911 Gefunden im Internet: URL:http://onlinelibrary.wiley.com/doi/10. 1002/chem.200801911/full>
- RURACK K ET AL: "A highly efficient sensor molecule emitting in the near infrared (NIR): 3,5-distyryl-8-(p-dimethylaminophenyl)-dif luoroboradiaza-s-indace", NEW JOURNAL OF CHEMISTRY, ROYAL SOCIETY OF CHEMISTRY, GB, Bd. 25, Nr. 2, 1. Januar 2001 (2001-01-01) , Seiten 289-292, XP002482384, ISSN: 1144-0546, DOI: DOI:10.1039/B007379M
- DOST Z ET AL: "Distyryl-boradiazaindacenes: facile synthesis of novel near IR emitting fluorophores", TETRAHEDRON, ELSEVIER SCIENCE PUBLISHERS, AMSTERDAM, NL, Bd. 62, Nr. 36, 4. September 2006 (2006-09-04), Seiten 8484-8488, XP025002582, ISSN: 0040-4020, DOI: DOI:10.1016/J.TET.2006.06.082 [gefunden am 2006-09-04]
- O.R GALANGAU, C. DUMAS-VERDES, R. MÉALLET-RENAULT, G. CLAVIER: "Rational design of visible and NIR distyryl-BODIPY dyes from a novel fluorinated platform", ORGANIC & BIOMOLECULAR CHEMISTRY, Bd. 8, 17. August 2010 (2010-08-17), Seiten 4546-4553, XP002621753, DOI: 10.1039/C004812G Gefunden im Internet: URL:http://pubs.rsc.org/en/Content/Article Landing/2010/OB/c004812g>

## Beschreibung

### Technologischer Hintergrund und Stand der Technik

Photostabile Farbstoffe, die eine nennenswerte bis stark ausgeprägte Absorption im gesamten sichtbaren Spektralbereich, eine moderate bis hohe Fluoreszenzquantenausbeute und eine große Stokes'sche Verschiebung im nahen Infrarot (NIR)-Spektralbereich aufweisen, nur schwach solvatochrom und netto ungeladen (d.h. neutral oder zwitterionisch) sind, stellen geeignete Markierungs- oder Referenzmaterialien in chemischen, physikalischen oder biologischen Anwendungen dar, die auf der Messung von Fluoreszenzsignalen beruhen.

Heute erhältliche NIR-Farbstoffe sind entweder gekennzeichnet durch
(i) schmale und ausgeprägte Absorptionsbanden im NIR, die eine Anregung und Verwendung im sichtbaren Bereich ausschließen, mit schmalen Fluoreszenzbanden und geringen Stokes'schen Verschiebungen, nur niedrigen bis moderaten Fluoreszenzquantenausbeuten und geringer Photostabilität (z.B. Cyanin-Farbstoffe),
(ii) durch breite Absorptionsbanden aber nur gering Stokes-verschobene und schmale Fluoreszenzbanden mit geringen Fluoreszenzquantenausbeuten, die nur schwache Signale liefern (z.B. Porphyrine),
(iii) durch breite Absorptions- und stark Stokes-verschobene Fluoreszenzbanden aber nur geringe Fluoreszenzquantenausbeuten und eine große Solvatochromie, d.h. eine hohe Empfindlichkeit gegenüber Änderungen in ihrer unmittelbaren Umgebung (z.B. Styryl- oder Oxazin-Farbstoffe),
(iv) durch schmale und ausgeprägte Absorptionsbanden im NIR, die eine Anregung und Verwendung im sichtbaren Bereich ausschließen, mit schmalen Fluoreszenzbanden und geringen Stokes'schen Verschiebungen, aber mit hohen Fluoreszenzquantenausbeuten und hoher Photostabilität (z.B. Terrylenimid-Farbstoffe),
(v) durch gute Photostabilität und breite Absorptions- und Emissionsbanden aber nur geringe Fluoreszenzquantenausbeuten (z.B. Quaterrylene-bis(dicarboximid)-Farbstoffe) oder
(vi) durch schmale und ausgeprägte Absorptionsbanden im NIR, die eine Anregung und Verwendung im sichtbaren Bereich ausschließen, mit geringen Stokes'schen Verschiebungen und einer Empfindlichkeit gegenüber nucleophilen Spezies (z.B. Squarain-Farbstoffe),

Zudem tragen die meisten der heute erhältlichen NIR-Farbstoffe, mit Ausnahme der Porphyrine, Squaraine und Perylen-Derivate, eine Nettoladung (meist eine positive), was ihre Verwendung auf polare Lösungsmittel, Materialien und Umgebungen beschränkt.

Hierdurch ergeben sich folgende Nachteile:
(i) schmale Absorptionsbanden = keine Möglichkeit für breitbandige Anregung und Einsatz,
(ii) geringe Stokes'sche Verschiebungen = nur ungenügende Trennung von Anregungslicht/Streuung und Fluoreszenzsignal möglich,
(iii) niedrige Fluoreszenzquantenausbeuten = schwache Fluoreszenzsignale,
(iv) geringe Photostabilität = ungenügende Lebenszeit des Farbstoffes oder Bauteils, und
(v) ausgeprägte Solvatochromie = unerwünschte Signalfluktuationen bei Änderungen der Umgebungsparameter.

Ziel der Erfindung ist es, elektronisch neutrale NIR-emittierende Farbstoffe mit besseren Eigenschaften, d.h. breitbandiger Anregungsmöglichkeit, intensiver NIR-Fluoreszenz, großer Stokes'scher Verschiebung, hoher Photostabilität, geringer Solvatochromie und einer Unempfindlichkeit gegenüber Umgebungseinflüssen als potente Fluorophore, Marker oder Referenzmaterialien für verschiedenste NIR-fluorometrische Anwendungen bereitzustellen.

In diesem Zusammenhang hat die Klasse der Difluorboryl-Komplexe des Dipyrrins (auch 4,4-Difluoro-4-bor-3a,4a-diaza-s-indacen) wegen der augenscheinlichen Fluoreszenz dieser Verbindungsklasse besondere Bedeutung. Vertreter dieser Verbindungsklasse werden unter dem Handelsnamen BODIPY (aus dem engl. für BOron DIPYrrin) als Fluoreszenzmarker für molekularbiologische Anwendungen vermarktet. Neben seinem ursprünglichen Einsatz als Biolabel findet der Farbstoff auch als Kationen-Sensor, Laserfarbstoff und in anderen Bereichen der Materialwissenschaften Anwendung. Die Farbstoffe sind mit variablem Substitutionsmuster synthetisch relativ einfach und in präparativen Mengen zugänglich. Die Ursache für die vielseitige und überaus erfolgreiche Einsatzbreite dieser Farbstoffklasse lässt sich auf einige grundlegende Eigenschaften zurückführen, wie hoher molarer Extinktionskoeffizient (ε > 80.000 M⁻¹cm⁻¹), hohe Fluoreszenzquantenausbeute (Φ > 0.70) und moderates Redoxpotential. Vertreter dieser Farbstoffklasse lassen sich beispielsweise US 5,248,782; US 6,005,113; E. Deniz et al., Organic Letters (2008), Vol. 10, Nr. 16, Seiten 3401 - 3403; S. Erten-Ela et al., Organic Letters (2008), Vol. 10, Nr. 15, Seiten 3299 - 3302; O. Zheng et al., Chem. Eur. J. (2008), Nr. 14, Seiten 5812 - 5819; und Ziessel et al. , Chem. Eur. J. (2009), Nr. 15, Seiten 1359 - 1369 entnehmen.

Als Nachteil erweist sich, das der BODIPY-Farbstoff klassischerweise auf den Einsatz im Wellenlängenbereich von 470 bis 530 nm beschränkt ist. Zusätzlich ist eine Stokes'sche Verschiebung von 5 bis 15 nm für viele Anwendungen ein limitierender Faktor. Insbesondere für die Verwendung in der Einzelmolekülspektroskopie und in Multiplexing-Anwendungen ist diese Eigenschaft ein drastischer Nachteil.

### Zusammenfassung der Erfindung

Durch die Erfindung sollen eines oder mehrere der vorgenannten Probleme gelöst oder zumindest gemindert werden. Dazu stellt die Erfindung die nachfolgenden Difluoroboradiazaindacen-Farbstoffe der Formel (1) bereit: 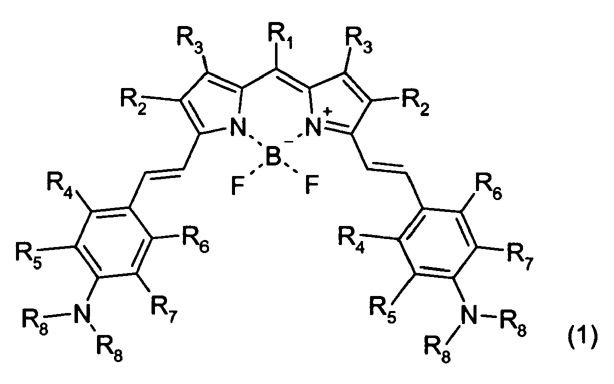 mit
R₁ = Fluor-substituierter Phenylrest C₆HₘFₙ mit n = 1 bis 5 und m+n = 5; oder Fluor-substituierter Naphthylrest C₁₀HₘFₙ mit n = 1 bis 9 und m+n = 9;
R₂ = CH₃, C₂H₅, C₃H₇ oder C₄H₉;
R₃ = Alkyl, Aryl oder Vinylaryl;
R₄, R₅ = H, F oder einen R₄ und R₅ verbrückenden Rest CH=CH-CH=CH;
R₆, R₇ = H, F oder einen R₆ und R₇ verbrückenden Rest CH=CH-CH=CH; und
R₈ = Alkyl oder Aryl.

Vorzugsweise steht R₁ für C₆F₅.

Weiterhin ist bevorzugt, wenn R₃ für CH₃ steht, insbesondere auch in Kombination mit den zuvor beschriebenen bevorzugten Ausführungsformen.

Ferner ist bevorzugt, wenn R₄ und R₅ ein verbrückender Rest CH=CH-CH=CH ist, insbesondere auch in Kombination mit den zuvor beschriebenen bevorzugten Ausführungsformen.

Besonders bevorzugt sind Difluoroboradiazaindacen-Farbstoffe der Formeln (2) und (3):

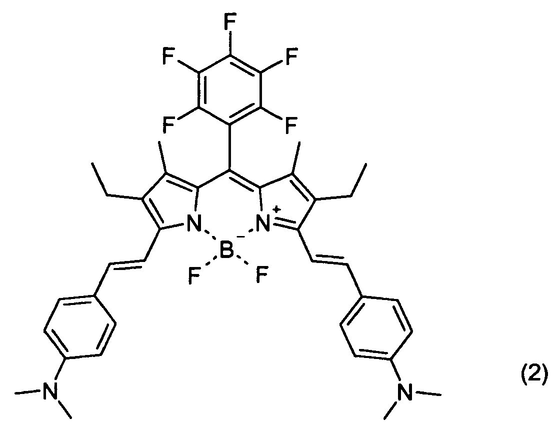

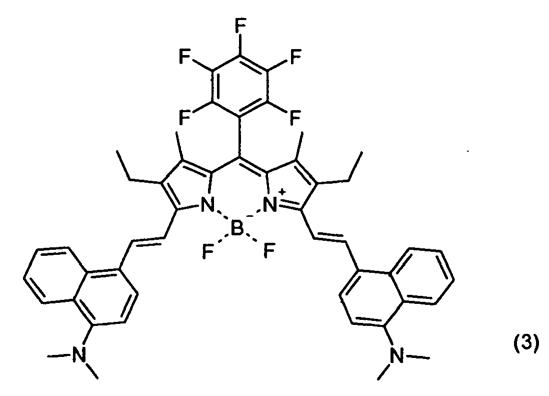

Die Farbstoffe sind in der Regel in un-, mittel- und hoch polaren Umgebungen und Medien, egal ob gelöst in molekularer Form oder eingekapselt in Partikeln oder anderen Materialien, einsetzbar.

### Detaillierte Beschreibung der Erfindung

Die Erfindung wird nachfolgend anhand von zwei Ausführungsbeispielen **dnBDP5F** (entspricht Formel (3)) und **dsBDP5F** (entspricht Formel (2)) und zugehöriger Vorstufe **BDP5F** (siehe Schema 1) näher erläutert.

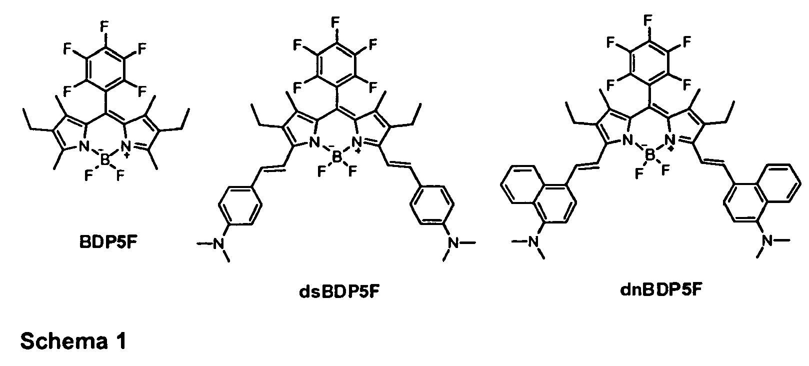

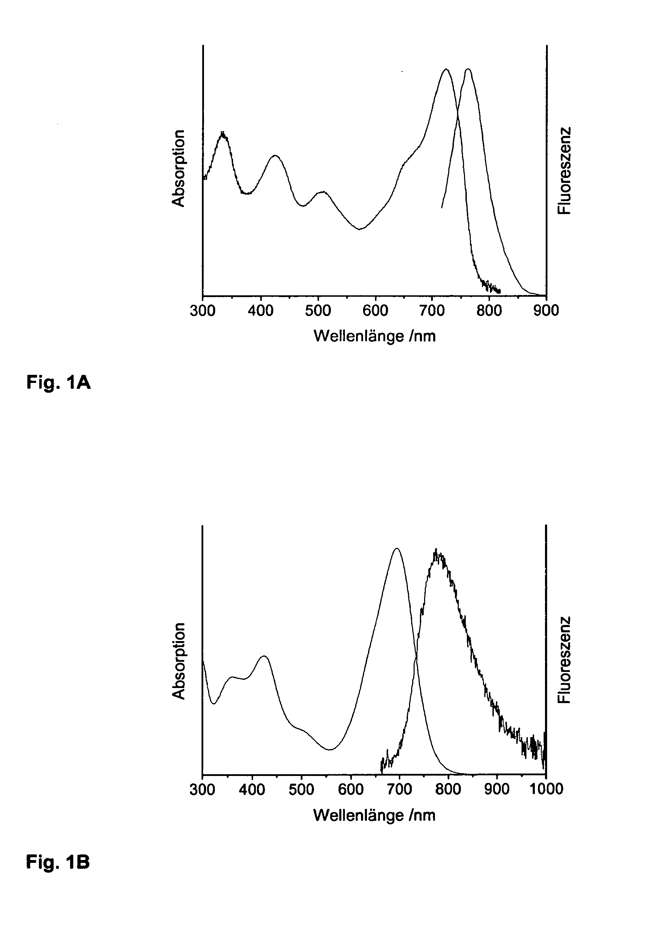

Die Figuren 1A und 1B zeigen Absorptions- und Fluoreszenzspektren der Farbstoffe **dnBDP5F** und **dsBDP5F** in Diethylether.

### Experimenteller Teil

Alle Reagenzien stammen von kommerziellen Herstellern und wurden ohne weitere Aufreinigung eingesetzt, sofern nicht anders angegeben. Alle luft- und feuchtigkeitsempfindlichen Reaktionen wurden unter Argon-Atmosphäre in trockenen Glasapparaturen durchgeführt. Dichlormethan wurde über Calciumhydrid destilliert und Triethylamin durch Destillation aufgereinigt. Die NMR-Spektren wurden mit 400 und 600 MHz Geräten von Bruker gemessen.

### Herstellung der Vorstufe BDP5F

3-Ethyl-2,4-dimethylpyrrol (369.6 mg, 3 mmol) und 2,3,4,5,6-Pentafluorbenzaldehyd (353.0 mg, 1.8 mmol) wurden in trockenem CH₂Cl₂ (70 mL) unter Argon-Atmosphäre gelöst. Ein Tropfen Trifluoressigsäure (TFA) wurde zugesetzt und die Lösung wurde im Dunkeln für 5h bei Raumtemperatur gerührt. 2,3-Dichlor-5,6-dicyano-1,4-benzoquinon (DDQ, 408.6 mg, 1.8 mmol) wurden zugesetzt und das Gemisch für weitere 30 min gerührt. Das Reaktionsgemisch wurde dann mit Triethylamin (4 mL) und Bortrifluoridethyletherat (4 mL) versetzt. Nach weiteren 30 min Rühren wurde die tiefrote Lösung mit Wasser (3 × 50 mL) und Dichlormethan (50 mL) gewaschen, über Na₂SO₄ getrocknet und unter vermindertem Druck aufkonzentriert. Das Rohprodukt wurde durch Säulenchromatographie an Silicagel (50 % v/v CH₂Cl₂/Hexan) aufgereinigt und aus Hexan umkristallisiert. Es wurden orangerote Kristalle erhalten (166 mg; 20% Ausbeute).
NMR: ¹H-NMR (400 MHz, CDCl₃) [ppm] δ = 2.55 (s, 6H, CH₃), 2.34 (q, *J* = 7.6 Hz, 4H, CH₂), 1.51 (s, 6H, CH₃), 1.02 (t, *J* = 7.6 Hz, 6H, CH₃).

### Herstellung von dnBDP5F und dsBDP5F:

Generell wurden die entsprechenden Aldehyde und **BDP5F** unter Rückfluss für 26 h in einem Lösungsmittelgemisch aus trockenem Toluen (5 mL), Eisessig (0.15 mL), und Piperidin (0.18 mL) mit einem geringen Zusatz von 4-Å Molekularsieb erhitzt. Nach dem Abkühlen auf Raumtemperatur wurde das Lösungsmittel unter Vakuum entzogen und das Rohprodukt über eine Silica-Säule mit 50% v/v CH₂Cl₂/Hexan eluiert.

**dsBDP5F:** 19.2 mg (0.13 mmol) 4-Dimethylaminbenzaldehyd und 30 mg (0.06 mmol) **BDP5F** lieferten **dsBDP5F** als ein braunes Pulver. Ausbeute 2 %.
¹H-NMR (600 MHz, CDCl₃) [ppm] δ = 7.63 (d, *J* = 16.7 Hz, 2H, CH), 7.54 (d, *J* = 8.9 Hz, 4H, CH), 7.23 (d, *J* = 16.7 Hz, 2H, CH), 6.74 (d, *J* = 8.9 Hz, 4H, CH), 3.03 (s, 12H, CH₃), 2.64 (q, *J* = 7.6 Hz, 4H, CH₂), 1.57 (s, 6H, CH₃), 1.19 (t, *J* = 7.6 Hz, 6H, CH₃).

**dnBDP5F:** 37.3 mg (0.187 mmol) 4-(Dimethylamin)-1-naphthaldehyd und 38.3 mg (0.08 mmol) **BDP5F** lieferten **dnBDP5F** als grünen Feststoff. Ausbeute 1 %.
¹H-NMR (600 MHz, CDCl₃) [ppm] δ = 8.26 (m, 2H, CH), 8.17 (m, 2H, CH), 8.15 (d, *J* = 16.9 Hz, 2H, CH), 7.96 (d, *J* = 7.9 Hz, 2H, CH), 7.80 (d, *J* = 16.4 Hz, 2H, CH), 7.52 (m, 4H, CH), 7.16 (d, *J* = 7.9 Hz, 2H, CH), 2.95 (s, 12H, CH₃), 2.75 (q, *J* = 7.5 Hz, 4H, CH₂), 1.65 (s, 6H, CH₃), 1.31 (t, *J* = 7.5 Hz, 6H, CH₃).

### Untersuchungen zur Photostabilität

Untersuchungen zur Photostabilität erfolgten unter Zuhilfenahme einer 150 W Xenon Lampe, mit der eine 1 mm Küvette bei 670 nm mit einer Spaltbreite von 15 nm bestrahlt wurde. Der Lichtstrahl wurde auf einen 1.5 cm² große Fläche der Küvette fokussiert. Die Transmission wurde durch einen 600 nm Filter erfasst. 300 µL Farbstofflösung mit einer Konzentration von 1.8 × 10⁻⁵ mmol L⁻¹ **(dnBDP5F)** und 3.5 × 10⁻⁵ mmol L⁻¹ **(dsBDP5F)** in THF, entsprechend optischen Dichten von 0.08 bzw. 0.05, wurden als Proben verwendet. Die Bestrahlung wurde auf 17 h mit einer durchschnittlichen Bestrahlungsintensität von 0.5 mW cm⁻² begrenzt. Die Bestrahlungsenergie wurde mit einer kalibrierten Si-Diode gemessen, um die Anzahl transmittierter und absorbierter Photonen zu bestimmen. Auf Basis der Messungen wurde der Abfall der Konzentration nach dem Lambert-Beer'schen-Gesetz berechnet.

Repräsentative Spektren und spektroskopische Daten von **dsBDP5F** und **dnBDP5F** sind in den Tabellen 1 und 2 und Figuren 1A und 1B zusammengestellt.

Spektroskopische Daten von **dsBDP5F** in verschiedenen Lösungsmitteln bei 298 K, einer Farbstoffkonzentration von 2 × 10⁻⁶ M und unter Anregung bei λₑₓ = 670 nm (n.s. = nicht ausreichend löslich):

**Tabelle 1**

| Lösungsmittel | λ_{abs} /nm | λₑₘ /nm | Δν_{abs-em}/cm⁻¹ | Φ_{f} |
|---|---|---|---|---|
| Hexan | n.s. | n.s. | - | - |
| Dibutylether | 723 | 763 | 750 | 0.43 |
| Diethylether | 723 | 772 | 890 | 0.18 |
| THF | 739 | 792 | 900 | 0.11 |

Spektroskopische Daten von **dnBDP5F** in verschiedenen Lösungsmitteln bei 298 K, einer Farbstoffkonzentration von 2 × 10⁻⁶ M und unter Anregung bei λₑₓ = 640 nm:

**Tabelle 2**

| Lösungsmittel | λ_{abs} /nm | λₑₘ /nm | Δν_{abs}-em/cm⁻¹ | Φ_{f} |
|---|---|---|---|---|
| Hexan | 692 | 742 | 980 | 0.23 |
| Dibutylether | 697 | 766 | 1290 | 0.17 |
| Diethylether | 696 | 779 | 1530 | 0.13 |
| THF | 699 | 827 | 2210 | 0.06 |

Repräsentative molare Absorptionskoeffizienten und Angaben zur Photostabilität wurden für **dsBDP5F** und **dnBDP5F** in THF erhalten. Die hervorragende Photostabilität der Farbstoffe zeigt sich insbesondere durch den Abfall von nur 1 % für **dsBDP5F** und 2 % für **dnBDP5F** der Farbstoffabsorption nach 17 h Bestrahlung. Es wird angenommen, dass der Effekt unter anderem durch die Verwendung des Pentafluorphenyl-Restes in meso-Position bedingt ist. Tabelle 3 vergleicht die spektroskopischen Daten von **BDP5F** und seinem Phenylanalogon, 1,3,5,7-Tetramethyl-2,6-diethyl-8-phenyl-4-difluorobora-3a,4a-diaza-(s)-indacen (**BDP5H**) in ausgewählten Lösungsmitteln bei 298 K, einer Farbstoffkonzentration von 2 × 10⁻⁶ M und unter Anregung bei λₑₓ = 500 nm.

**Tabelle 3**

| | Lösungsmittel | λ_{abs} /nm | λₑₘ /nm | Δν_{abs-em}/cm⁻¹ | Φ_{f} |
|---|---|---|---|---|---|
| BDP5F | Dibutylether | 543 | 557 | 460 | 0.95 |
| | Diethylether | 541 | 557 | 530 | 1.00 |
| BDP5H | Dibutylether | 524 | 535 | 390 | 0.78 |
| | Diethylether | 522 | 535 | 460 | 0.77 |

## Patentansprüche

1. Difluoroboradiazaindacen-Farbstoff der Formel (1) 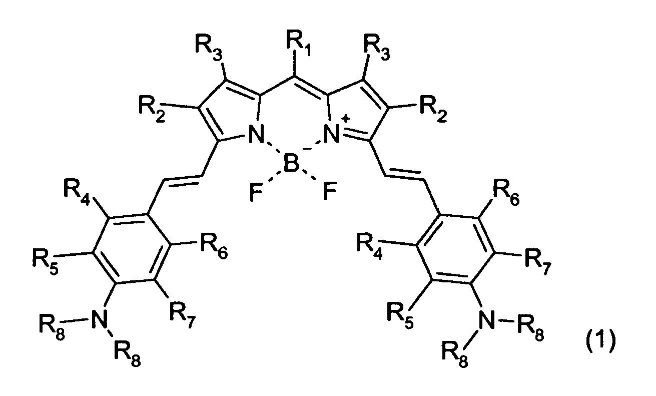 mit
R₁ = Fluor-substituierter Phenylrest C₆HₘFₙ mit n = 1 bis 5 und m+n = 5; oder Fluor-substituierter Naphthylrest C₁₀HₘFₙ mit n = 1 bis 9 und m+n = 9;
R₂ = CH₃, C₂H₅, C₃H₇ oder C₄H₉;
R₃ = Alkyl, Aryl oder Vinylaryl;
R₄, R₅ = H, F oder einen R₄ und R₅ verbrückenden Rest CH=CH-CH=CH;
R₆, R₇ = H, F oder einen R₆ und R₇ verbrückenden Rest CH=CH-CH=CH; und
R₈ = Alkyl oder Aryl.

2. Difluoroboradiazaindacen-Farbstoff nach Anspruch 1, bei dem
R₁ für C₆F₅ steht.

3. Difluoroboradiazaindacen-Farbstoff nach einem der vorhergehenden Ansprüche, bei dem
R₃ für CH₃ steht.

4. Difluoroboradiazaindacen-Farbstoff nach einem der vorhergehenden Ansprüche, bei dem
R₄ und R₅ ein verbrückender Rest CH=CH-CH=CH ist.

5. Difluoroboradiazaindacen-Farbstoff der Formel (2)

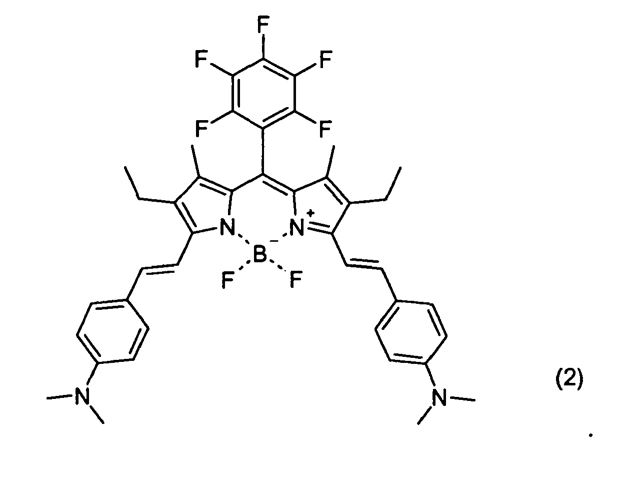

6. Difluoroboradiazaindacen-Farbstoff der Formel (3)

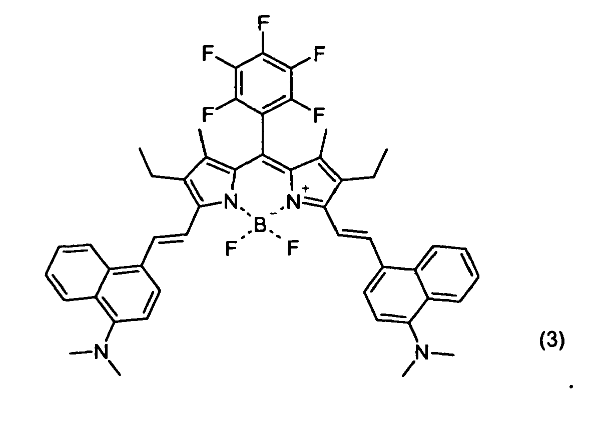

## Claims

1. A difluoroboradiazaindacene dye with formula (1): 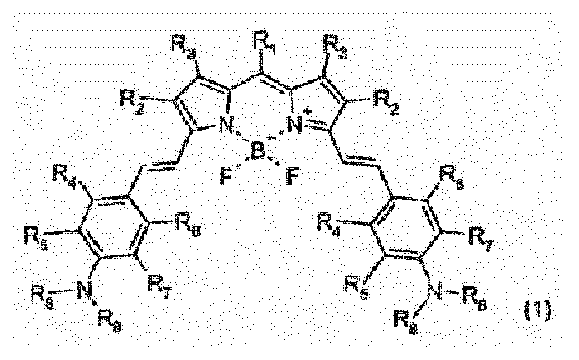 in which:
R₁ = a fluoro-substituted phenyl residue C₆HₘFₙ, in which n = 1 to 5 and m + n = 5; or a fluoro-substituted naphthyl residue C₁₀HₘFₙ, in which n = 1 to 9 and m + n = 9;
R₂ = CH₃, C₂H₅, C₃H₇ or C₄H₉;
R₃ = alkyl, aryl or vinylaryl;
R₄, R₅ = H, F or a residue CH=CH-CH=CH bridging R₄ and R₅;
R₆, R₇ = H, F or a residue CH=CH-CH=CH bridging R₆ and R₇; and
R₈ = alkyl or aryl.

2. The difluoroboradiazaindacene dye as claimed in claim 1, wherein
R₁ represents C₆H₅.

3. The difluoroboradiazaindacene dye as claimed in one of the preceding claims, wherein:
R₃ represents CH₃.

4. The difluoroboradiazaindacene dye as claimed in one of the preceding claims, wherein:
R₄ and R₅ represent a bridging residue CH=CH-CH=CH.

5. A difluoroboradiazaindacene dye with formula (2):

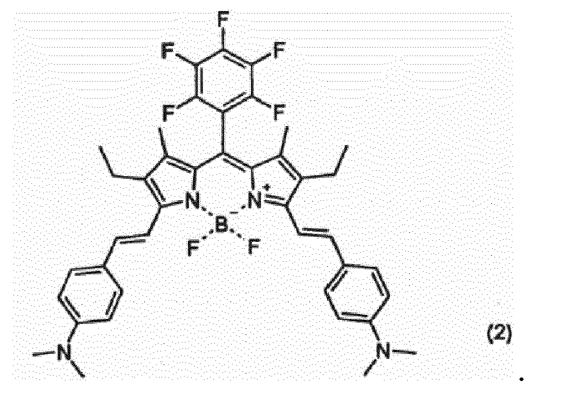

6. A difluoroboradiazaindacene dye with formula (3):

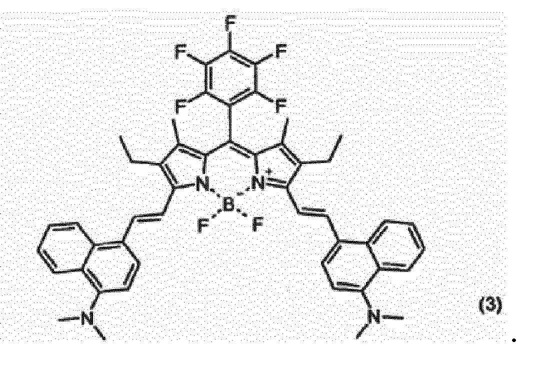

## Revendications

1. Colorant de difluoroboradiazaindacène de la formule (1) 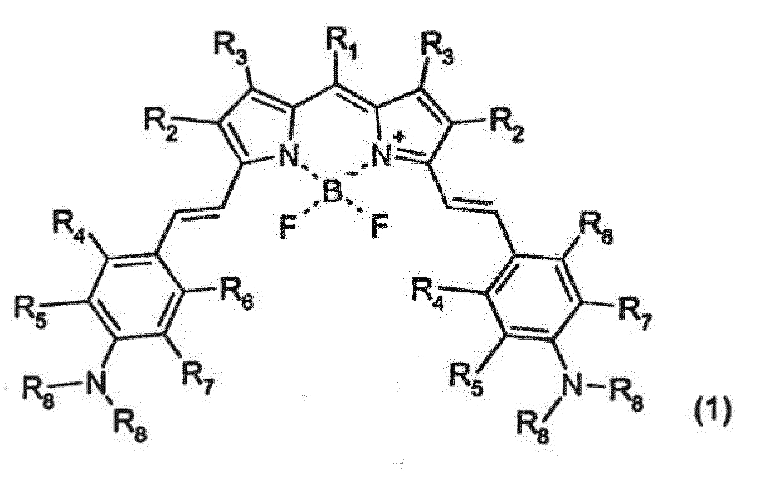 avec
R₁ = un radical phényle C₆HₘFₙ substitué par fluor, avec n = de 1 à 5 et m + n = 5 ; ou un radical naphtyle C₁₀HₘFₙ substitué par fluor, avec n = de 1 à 9 et m + n = 9 ;
R₂ = CH₃, C₂H₅, C₃H₇ ou C₄H₉ ;
R₃ = un alkyle, un aryle ou un vinylaryle
R₄, R₅ = H, F ou un radical CH=CH-CH=CH pontant R₆ et R₇ ;
R₆, R₇ = H, F ou un radical CH=CH-CH=CH pontant R₆ et R₇ ; et
R₈ = un alkyle ou un aryle.

2. Colorant de difluoroboradiazaindacène selon la revendication 1, dans lequel
R₁ est écrit pour C₆F₅.

3. Colorant de difluoroboradiazaindacène selon l'une quelconque des revendications précédentes, dans lequel
R₃ est écrit pour CH₃.

4. Colorant de difluoroboradiazaindacène selon l'une quelconque des revendications précédentes, dans lequel
R₄ et R₅ est un radical pontant CH=CH-CH=CH.

5. Colorant de difluoroboradiazaindacène de la formule (2)

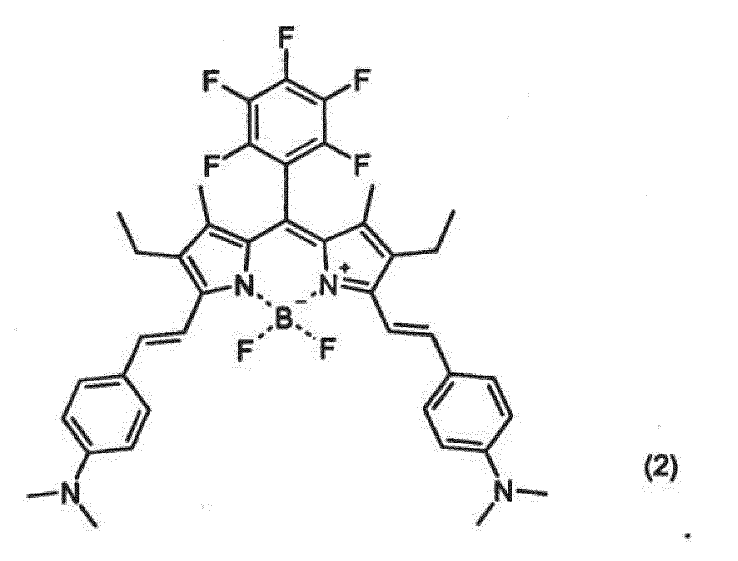

6. Colorant de difluoroboradiazaindacène de la formule (3)
